(19) Europäisches Patentamt
European Patent Office
Office européen des brevets

(11) Publication number: **0 487 514 B1**

(12) **EUROPEAN PATENT SPECIFICATION**

(45) Date of publication of patent specification: **14.06.95**  (51) Int. Cl.⁶: **C07C 209/16**

(21) Application number: **92200340.5**

(22) Date of filing: **07.10.88**

(60) Publication number of the earlier application in accordance with Art.76 EPC: **0 312 253**

(54) **Process for preparing N-substituted amine.**

(30) Priority: **16.10.87 JP 261366/87**
     **16.10.87 JP 261367/87**
     **16.10.87 JP 261368/87**
     **16.10.87 JP 261369/87**

(43) Date of publication of application:
     **27.05.92 Bulletin 92/22**

(45) Publication of the grant of the patent:
     **14.06.95 Bulletin 95/24**

(84) Designated Contracting States:
     **DE ES**

(56) References cited:
     **DE-A- 3 034 433**
     **DE-A- 3 641 666**
     **US-A- 4 625 063**

     **WORLD PATENTS INDEX LATEST Derwent Publications Ltd., London, GB; AN 84-191036 & JP-A-59 106 441 (NEW JAPAN CHEM)**

(73) Proprietor: **KAO CORPORATION**
     **14-10, Nihonbashi Kayaba-cho 1-chome**
     **Chuo-ku**
     **Tokyo (JP)**

(72) Inventor: **Abe, Hiroshi**
     **1450 Nishihama**
     **Wakayama-shi,**
     **Wakayama (JP)**
     Inventor: **Aikawa, Jun**
     **1130, Nishihama**
     **Wakayama-shi,**
     **Wakayama (JP)**
     Inventor: **Okabe, Kazuhiko**
     **283-28, Hatage,**
     **Iwadecho**
     **Naga-gun,**
     **Wakayama (JP)**
     Inventor: **Sotoya, Kohshiro**
     **446-26, Nakaguro,**
     **Iwadecho**
     **Naga-gun,**
     **Wakayama (JP)**

(74) Representative: **Bannerman, David Gardner et al**
     **Withers & Rogers**
     **4 Dyer's Buildings**
     **Holborn**
     **London, EC1N 2JT (GB)**

## Description

The present invention relates to a process for preparing an N-substituted amine which comprises reacting an alcohol or an aldehyde with ammonia or a primary or secondary amine.

The amine prepared according to the present invention is a material important from the industrial viewpoint as intermediates of a rust preventive, a surfactant, a germicide, a dyeing assistant and a fiber softener, etc.

[Prior Art]

It is known in the art that an alcohol or an aldehyde is reacted with ammonia or a primary or secondary amine to give the corresponding amine. However, it was difficult to selectively prepare a particular amine through a reaction of an alcohol or the like with an amine or the like.

Examples of the process for preparing an amine from an alcohol and an amine include those disclosed in Japanese Patent Laid-Open Nos. 19604/1977 (a copper chromite catalyst and a cobalt catalyst) and 59602/1978 (copper/molybdenum and copper/tungsten catalysts), U.S. Patent No. 3 223 734 (Raney nickel catalyst and copper chromite catalysts), West German Patent Laid-Open No. 1,493,781 (a supported cobalt catalyst), Japanese Patent Publication No. 55704/1982 (a copper/nickel catalyst), etc. However, these catalysts are insufficient in both the catalytic activity and the selectivity and should be used in a large amount, which brings about a low yield of the intended amine. In order to solve these problems, processes described in Japanese Patent Laid-Open Nos. 15865/1986, 149646/1987, 149647/1987, and 149648/1987 have been developed. In these processes, a copper/nickel/group VIII platinum metal element catalyst is used to prepare an intended amine in a high yield. That is, in these processes, an intended amine is prepared in a high yield through the addition of a small amount of the group VIII platinum metal element to a conventional copper/nickel catalyst having insufficient activity and selectivity for the purpose of improving the activity and selectivity.

However, the process in which the reaction is conducted in the presence of this catalyst is not always satisfactory from the viewpoint of the durability of the catalyst. Specifically, although this process is superior in the activity and the selectivity to other general processes and brings about little or no lowering in the activity of the catalyst even after recovery and reuse of tens of times, the selectivity is lowered. Therefore, with the consideration of the preparation of an N-substituted amine on a commercial scale, a further improvement in the durability is desired from the viewpoint of the yield and quality of the formed N-substituted amine.

However, N-substituted amines which have been prepared in the presence of these catalysts bring about the deterioration of hue when converted into a quaternary ammonium salt (tetraalkylammonium salt, trialkylbenzylammonium salt, etc.), which unfavorably causes remarkable deterioration of performance in applications such as surfactants.

( Summary of the Invention )

The invention provides a process for preparing an N-substituted amine, which comprises the step of reacting an alcohol or an aldehyde with ammonia, a primary amine or a secondary amine at 150 to 250°c at a pressure of the atmospheric pressure to 101.325 bar (100 atm gauge), while water formed in the reaction is being removed out, in the presence of a catalyst comprising:

(1) copper; (2) cobalt; and (3) a metal of the platinum VIII group.

It is preferable that a molar ratio of the component (1) to (2) ranges from 10/90 to 99/1, preferably 1/9 to 9/1 or 50/50 to 99/1, a molar ratio of the component (3) to the sum total of (1) and (2) ranges from 0.001 to 0.1 and a molar ratio of the component (2) to (4) ranges from 1/0.01 to 1/1.

The invention will be explained in detail with reference to the catalyst.

When a copper/cobalt/group VIII platinum metal element catalyst prepared by adding a small amount of a group VIII platinum metal element to a copper/cobalt catalyst is used for an amination reaction, an N-substituted amine can be prepared with activity and selectivity equal or superior to those of a copper/nickel/group VIII platinum metal element catalyst and that the synthesis of a quaternary ammonium salt from the N-substituted amine prepared in the presence of this catalyst brings about little or no deterioration of hue. Platinum, palladium, ruthenium, and rhodium were effective as the group VIII platinum metal element.

Consequently, the use of cobalt instead of nickel in the copper/nickel/group VIII platinum metal element catalyst enabled the development of a high performance amination catalyst not only having activity and

2

selectivity equal or superior to those of the copper/nickel/group VIII platinum metal element catalyst but also capable of providing a high quality N-substituted amine causing little or no deterioration of hue when converted into a quaternary ammonium salt.

The catalyst of the present invention exhibits activity several times higher than that of the copper/nickel catalyst described in Japanese Patent Publication No. 55704/1982 as well as excellent selectivity of the reaction and has activity and selectivity equal or superior to that of the copper/nickel/group VIII platinum metal element catalyst. Further, as opposed to the copper/nickel/group VIII platinum metal element catalyst, the catalyst of the present invention brings about little or no deterioration of hue when the N-substituted amine is converted into a quaternary ammonium salt.

Although the catalyst metal composition should contain three components, i.e., copper, cobalt, and a platinum group element, the catalyst may also contain other fourth period transition metal elements in such a small amount as will not spoil the catalytic performance. Further, the catalyst suitable for the present invention may have various forms.

The metals suitable for the process of the present invention may have any of the following forms as far as the three metal components indispensable to the present invention bring about interaction thereamong through reduction in a hydrogen atmosphere:

1) a form of a metal, an oxide or a hydroxide thereof and a mixture thereof which can be dispersed in a reaction medium;

2) a form of either a mixture of copper, a fourth period transition metal element and a platinum group element respectively supported on suitable carriers, or three components, i.e., copper, a fourth period transition metal element and a platinum group element, homogeneously supported on a single carrier which can be dispersed in a reaction medium;

3) a form of an aliphatic carboxylic acid salt of these metals, a complex of these metals stabilized by a suitable ligand or the like which is converted into a metallic colloid in a reaction medium to form a homogeneous system; and

4) a mixture of a form described in the above items 1) and 2) which is dispersed in a reaction medium with a form described in the above item 3) which forms a homogeneous system in a reaction system, or a form which is in a dispersed state before hydrogen reduction but becomes homogeneous after hydrogen reduction.

With respect to the form of the catalyst used in the process of the present invention, it is preferred from the viewpoint of the stabilization of the catalyst metal, i.e., immobilization of the active surface, and resistance to a catalyst poison that the above-described three metal components are homogeneously supported on a suitable carrier.

When the three metal components according to the present invention, i.e., copper, cobalt and a platinum group VIII element, are to be supported on a carrier, suitable carriers are those commonly employed as catalyst carriers, e.g., alumina, silica/alumina, diatomaceous earth, silica, active carbon, and natural and artificial zeolite. Although the amount of the catalyst metal supported on a carrier may be arbitrarily determined, it is generally preferably 5 to 70 %.

Further, these three metal components may be supported on a carrier by various methods. In this case, the form of the raw metals of the catalyst may be an oxide, a hydroxide, or various salts of copper, a fourth period transition metal element, and a platinum group element. Examples of the form of the raw metals include chlorides, sulfates, nitrates, acetates, aliphatic carboxylates of copper, a fourth period transition metal element, and a platinum group element, or complexes of these metals, e.g., acetylacetone complexes and dimethylglyoxime complexes of copper, cobalt metal element, and a platinum group VIII element. Further, with respect to the platinum group element, carbonyl complexes, amine complexes, phosphine complexes, etc. may also be employed. The preparation of a catalyst through supporting of the metal components on a carrier by making use of these raw metal materials may be conducted by any of conventional known processes, e.g., a process which comprises adding a carrier to a solution of suitable salts of copper, cobalt and a platinum group VIII element to sufficiently impregnate the carrier with the solution and drying and baking the impregnated carrier (impregnation process), a process which comprises sufficiently mixing a carrier with an aqueous solution of suitable salts of copper, a fourth period transition metal element and a platinum group element and adding an aqueous alkaline solution, such as an aqueous sodium carbonate or sodium hydroxide solution or aqueous ammonia, to the mixture to precipitate the metal salts on the carrier, a process which comprises simultaneously adding an aqueous solution of suitable salts of copper, cobalt and a platinum group VIII element and an aqueous alkaline solution, such as an aqueous sodium carbonate or sodium hydroxide solution or aqueous ammonia, to a water slurry of a carrier in such a manner that the pH value of the slurry remains constant (e.g., a constant pH value of 7) to precipitate the metal salts on the carrier (the above two processes are called "coprecipitation process"), a process which comprises ion exchanging sodium, potassium or the like with copper, cobalt and a platinum group VIII element on zeolite (ion exchange process), and a process which comprises heat melting copper, cobalt, a platinum group VIII element and an aluminum metal, cooling the resulting melt to form an alloy and eluting aluminum contained in the alloy with an alkali such as sodium hydroxide (alloy process). In the case of the coprecipitation process, the carrier is sufficiently washed with water after deposition of the metals, dried at about 100°C, and baked at 300 to 700°C to prepare a catalyst.

Another effective process comprises supporting only copper or only copper and cobalt on a carrier through the above-described processes and adding cobalt and a platinum group element VIII or a supported platinum group VIII element prior to the reaction, or an aliphatic carboxylate or a complex thereof to form a composite comprising a combination of copper with the cobalt element and the platinum group VIII element in a reaction medium in a hydrogen atmosphere.

With respect to the form of the catalyst, it is more preferable that the three components be homogeneously supported on the same carrier.

The three components, i.e., copper, cobalt and a platinum group element VIII, are indispensable to the present invention.

The alcohol or aldehyde which is a starting material used in the present invention is a straight-chain or branched saturated or unsaturated aliphatic alcohol or aldehyde having 8 to 36 carbon atoms. Examples of the alcohol include octyl alcohol, lauryl alcohol, myristyl alcohol, stearyl alcohol, behenyl alcohol, oleyl alcohol, and a mixture thereof, a Ziegler alcohol prepared by the Ziegler process, an oxo alcohol prepared by the oxo process, and alcohols having a branched chain such as Guerbet alcohol. Examples of the aldehyde include lauraldehyde, oxo aldehyde, and aldehydes corresponding to the above-described alcohols.

Further, various polyhydric alcohols may also be used, and examples thereof include 1,3-butanediol, 1,4-butanediol, 1,5-pentanediol, 1,6-hexanediol, 1,9-nonanediol, diethylene glycol, and triethylene glycol. Examples of other alcohol include aromatic alcohols, such as benzyl alcohol and phenethyl alcohol, polyoxy ether alcohols, such as adduct of an aliphatic alcohol with ethylene oxide or propylene oxide, and amino alcohols, such as ethanolamine and diethanolamine.

The alcohol or aldehyde is particularly preferably an aliphatic alcohol or aldehyde selected from among saturated and unsaturated straight-chain and branched aliphatic alcohols and aldehydes having 8 to 36 carbon atoms and aliphatic glycols having 2 to 12 carbon atoms. The amine which is reacted with these alcohols or aldehydes may be a gaseous or liquid one, and examples thereof include aliphatic amines, e.g., primary amines such as monomethylamine, ethylamine and dodecylamine, and secondary amines such as dimethylamine, diethylamine and didodecylamine.

In the present invention, it is necessary to remove water formed during a reaction of an alcohol or an aldehyde with an amine from the reaction system. When the formed water is not removed from the reaction system, no sufficient catalytic performance according to the present invention can be attained. That is, the catalytic activity and selectivity are lowered, so that it becomes difficult to easily prepare an N-substituted amine in a high yield. For example, when the reaction is conducted by making use of dimethylamine as the amine without removing the formed water, not only the amount of by-products, such as monoalkylmethylamine, which are difficult to separate through mere distillation is increased but also a high-boiling material, such as condensate of an aldehyde, is formed in a large amount, which brings about a lowering in the yield of the intended N-substituted amine.

Water may be intermittently or continuously removed during the reaction as far as the formed water is properly removed so as not to remain in the reaction system for a long period of time. It is preferred to continuously remove water each time it is formed. More specifically, it is a common practice to introduce a suitable amount of hydrogen gas into the reaction system to distill the formed water and the excess amine (in the case of use of a gaseous amine) together with the hydrogen gas. In this method, it is also possible to reuse the hydrogen gas through condensation and separation of the formed water in a condenser. Further, a suitable solvent may be added to the reaction system, followed by distillation and removal of the formed water in the form of an azeotrope with the solvent.

In the process of the present invention, although a catalyst which has separately been reduced with a hydrogen gas may be employed, a catalyst before reduction is placed in a reactor together with the starting material of the reaction, i.e., an alcohol or an aldehyde, followed by elevation of the temperature to the reaction temperature while introducing a hydrogen gas, thereby conducting reduction. That is, the copper/cobalt/platinum group VIII element catalyst according to the present invention has also a significant feature that since the reduction temperature is low, the reduction can be conducted in the step of elevating the temperature to the reaction temperature.

The preferred embodiments of the process of the present invention will now be briefly described.

4

A reaction vessel equipped with a tube for introducing hydrogen and an amine and a condenser and a separator for condensing and separating water formed during the reaction, the excess amine and oleaginous matter distilled off is charged with a starting material, i.e., an alcohol or an aldehyde, and a catalyst. The amount of the catalyst may be arbitrary. Since, however, the catalyst of the present invention has high activity, it is usually employed in an amount as small as 0.1 to 2 % by weight based on the feed alcohol or aldehyde. The system is purged with a nitrogen gas, and the temperature is then raised while introducing hydrogen. The reaction is usually conducted at a temperature of 150 to 250°c. However, the reaction temperature may be one outside the above-described range depending upon the kind of the reaction. The catalyst is converted into a reduced, active state during the elevation of the temperature. When the temperature reaches a predetermined value, ammonia or an amine is introduced to initiate the reaction. The amine may be gaseous or liquid. Further, the amine may be introduced into the system in a continuous or intermittent manner or at a time (in the case of a liquid amine). During the reaction, the formed water is discharged outside the reaction system together with gaseous substances (excess gaseous amine in the case of the use of hydrogen and a gaseous amine) and passed through the condenser and separator to separate the water from the oleaginous matter. The analysis of the gaseous substances (excess gaseous amine in the case of the use of hydrogen, and a gaseous amine) has revealed that these gaseous substances are substantially free from by-products (e.g., hydrocarbon and amine by-product formed by disproportionation of the starting amine), i.e., has proved that the catalyst of the present invention has high selectivity. Therefore, it was found that these gaseous substances can be reused without any special step of purification. After the completion of the reaction, the reaction mixture itself is subjected to distillation or filtration to separate the reactant from the catalyst. The N-substituted amine obtained by the filtration can be distilled into a very pure form.

Examples of Catalyst

Examples 1 and 2 and Comparative Examples 1 to 4

A copper/cobalt/platinum group element ternary catalyst supported on synthetic zeolite was prepared as follows.

A 1-ℓ flask was charged with synthetic zeolite, and an aqueous solution prepared by dissolving copper nitrate, cobalt nitrate and palladium chloride in water in a copper : cobalt : palladium molar ratio of 4 : 1 : 0.02 was then added thereto, followed by elevation of the temperature while stirring. An aqueous 10% $Na_2CO_3$ solution was gradually dropwise added thereto at 90°C. The mixture was aged for 1 hr, and the resultant precipitates were collected by filtration, washed with water, dried at 80°C for 10 hr, and baked at 400°C for 3 hr. The amount of the resultant metallic oxide supported on the carrier was 50%.

Then, a reaction of an alcohol with dimethylamine was conducted in the presence of the above-prepared catalyst. In Comparative Examples, the reaction was conducted in the presence of a copper/nickel/palladium catalyst and a copper/nickel catalyst prepared in the same manner as that described above.

A 1-ℓ flask equipped with a condenser and a separator for separating the formed water was charged with 600 g of lauryl alcohol and 1.5 g (0.25% by weight based on the starting alcohol) of the above-described catalyst. The system was purged with nitrogen while stirring, followed by initiation of the elevation of the temperature. When the temperature reached 100°C, a hydrogen gas was blown into the system through a flowmeter at a flow rate of 10 ℓ/hr, and the temperature was raised to 200°C. At this temperature, a mixed gas comprising dimethylamine and hydrogen was blown into the reaction system at a flow rate of 40 ℓ/hr, and the reaction was monitored by measurement of an amine value and gas chromatography.

The results are shown in Table 1.

Table 1

|  | catalyst composition molar ratio | reaction time (hr) | composition of reaction product (wt%) | | |
|---|---|---|---|---|---|
|  |  |  | unreacted alcohol | lauryldimethylamine | Others |
| Ex. 1 | Cu/Co/Pd = 4/1/0.02 | 5 | 1.4 | 95.5 | 3.1 |
| Comp. Ex. 1 | Cu/Ni = 4/1 | 10 | 5.3 | 89.0 | 5.7 |
| Comp. Ex. 2 | Cu/Ni/Pd = 4/1/0.02 | 5 | 1.6 | 92.4 | 6.0 |

It was found from the results that as with the Cu/Ni/platinum group element (Pd) ternary catalyst system (Comparative Example 2), the Cu/Co/platinum group element (Pd) ternary catalyst system according to the present invention brought about a reduction in the reaction time by half and high conversion of the starting alcohol by virtue of the incorporation of a small amount of a platinum group element, i.e., exhibited higher activity than that of the conventional Cu/Ni binary catalyst system (Comparative Example 1), by virtue of the incorporation of a small amount of a platinum group element.

Then, lauryldimethylamine prepared in the presence of the above-described catalysts was purified by distillation followed by a reaction with benzyl chloride or methyl chloride under ordinary conditions to synthesize a quaternary ammonium salt of lauryldimethylamine. The hue of the quaternary ammonium salt thus prepared was measured with Lovibond Red (by making use of a 1-in. cell).

The results are shown in Table 2.

Table 2

|  | * catalyst composition | Lovibond Red | |
|---|---|---|---|
|  |  | trimethyllaurylammonium chloride | dimethyllaurylbenzyl ammonium chloride |
| Ex. 2 | Cu/Co/Pd | 1.0 | 0.8 |
| Comp. Ex. 3 | Cu/Ni | 5.0 | 4.5 |
| Comp. Ex. 4 | Cu/Ni/Pd | 4.0 | 4.3 |

Note: * catalyst composition
Cu/Co/Pd = 4/1/0.02
Cu/Ni = 4/1
Cu/Ni/Pd = 4/1/0.02

It was found from the results that when the Cu/Co/platinum group element (Pd) ternary catalyst system according to the present invention was used, the hue of the quaternary ammonium salt was far better than that of the quaternary ammonium salt in the case where the Cu/Ni binary catalyst system (Comparative Example 3) and the Cu/Ni/Pd ternary catalyst system (Comparative Example 4) were used.

Examples 3 to 6 and Comparative Examples 5 and 6

A reaction of stearyl alcohol with monomethylamine was conducted in the presence of a copper/cobalt/platinum group element catalyst prepared in the same manner as that of Example 1. The reaction temperature was 200°C, and the amount of addition of the catalyst was 0.25% (based on alcohol). The tertiary amine thus prepared was purified by distillation, and the purified tertiary amine was reacted with benzyl chloride or methyl chloride to prepare a quaternary ammonium salt. In Comparative Examples, a copper/cobalt catalyst and a copper/nickel/platinum group element (Ru) catalyst were used.

The composition of the product in the reaction for preparation of the tertiary amine and the hue (Lovibond Red) of the corresponding quaternary ammonium salt are shown in Table 3.

6

Table 3

| | catalyst composition * | reaction time (hr) | composition of reaction product (wt%) | | | hue of quaternary ammonium salt (Lovibond Red) | |
|---|---|---|---|---|---|---|---|
| | | | unreacted alcohol | distearylmono-methylamine | Others | distearyl-dimethyl-ammonium chloride | distearyl-monomethyl-benzyl chloride |
| Ex. 3 | Cu/Co/Pd | 6 | 1.1 | 94.7 | 4.2 | 1.5 | 1.3 |
| Ex. 4 | Cu/Co/Pt | 6 | 1.5 | 94.8 | 3.9 | 2.0 | 1.8 |
| Ex. 5 | Cu/Co/Ru | 6 | 0.6 | 96.0 | 3.4 | 1.0 | 0.8 |
| Ex. 6 | Cu/Co/Rh | 6 | 1.3 | 94.9 | 3.8 | 1.8 | 1.6 |
| Comp. Ex. 5 | Cu/Co | 8 | 15.6 | 73.2 | 11.2 | 4.9 | 3.7 |
| Comp. Ex. 6 | Cu/Ni/Ru | 6 | 1.3 | 91.9 | 6.8 | 3.8 | 3.5 |

Note: * Cu/Co/Pd, Cu/Co/Pt, Cu/Co/Ru, and Cu/Co/Rh: molar ratio 5/2/0.02

Cu/Co: molar ratio 5/2

Cu/Ni/Ru: molar ratio 5/2/0.02

amount of support 50%

It was found from the results that when distearylmonomethyl tertiary amine was prepared through a reaction of stearyl alcohol with monomethylamine, the Cu/Co platinum group element catalyst exhibited high activity and high selectivity equal or superior to those of the Cu/Ni/Ru catalyst (Comparative Example 6) as opposed to the Cu/Co catalyst (Comparative Example 5). Further, it was found that when the reaction product was distilled and a quaternary ammonium salt was prepared from the formed tertiary amine, the use of the tertiary amine prepared in the presence of the Cu/Co/platinum group element catalyst as a starting material brought about a remarkable improvement in the hue over the case where the tertiary amine prepared in the presence or the conventional catalyst (Comparative Examples 5 and 6) was used as the starting material.

Examples 7 to 10 and Comparative Examples 7 and 8

A reaction of lauryl alcohol with ammonia was conducted in the presence of a copper/cobalt/platinum group element catalyst prepared in the same manner as that of Example 1. The ammonia feed rate was 10 ℓ/hr. The reaction temperature was 180°C, and the amount of addition of the catalyst was 1.0% (based on alcohol). The tertiary amine thus prepared was purified by distillation, and the purified tertiary amine was reacted with benzyl chloride or methyl chloride to prepare a quaternary ammonium salt. In Comparative Examples, a copper/cobalt catalyst and a copper/nickel/platinum group element (Ru) catalyst were used.

The composition of the product in the reaction for preparation of the tertiary amine and the hue (Lovibond Red) of the corresponding quaternary ammonium salt are shown in Table 4.

It was found from the results that when trilauryl tertiary amine was prepared through a reaction of lauryl alcohol with ammonium, the Cu/Co/platinum group element catalyst exhibited high activity and high selectivity equal or superior to those of the Cu/Ni/Ru catalyst (Comparative Example 8) as opposed to the Cu/Co catalyst (Comparative Example 7). Further, it was found that when the reaction product was distilled and a quaternary ammonium salt was prepared from the formed tertiary amine, the use of the tertiary amine prepared in the presence of the Cu/Co/platinum group element catalyst as a starting material brought about a remarkable improvement in

8

Table 4

| | | catalyst composition * | reaction time (hr) | composition of reaction product (wt%) | | | hue of quaternary ammonium salt (Lovibond Red) | |
|---|---|---|---|---|---|---|---|---|
| | | | | unreacted alcohol | trilauryl-amine | Others | trilauryl-monomethyl-ammonium chloride | trilauryl-benzyl-ammonium chloride |
| Ex. | 7 | Cu/Co/Pd | 10 | 1.1 | 96.7 | 2.2 | 1.7 | 1.6 |
| | 8 | Cu/Co/Pt | 10 | 1.5 | 95.6 | 2.9 | 2.3 | 1.8 |
| | 9 | Cu/Co/Ru | 10 | 2.6 | 95.0 | 2.4 | 0.9 | 0.9 |
| | 10 | Cu/Co/Rh | 10 | 1.3 | 95.9 | 2.8 | 2.3 | 1.9 |
| Comp. Ex. | 7 | Cu/Co | 10 | 33.6 | 47.2 | 19.2 | 5.4 | 3.9 |
| | 8 | Cu/Ni/Ru | 10 | 3.3 | 91.9 | 4.8 | 3.9 | 3.7 |

Note:  * Cu/Co/Pd, Cu/Co/Pt, Cu/Co/Ru, and Cu/Co/Rh:  molar ratio 4/1/0.005

Cu/Co:  molar ratio 4/1

Cu/Ni/Ru:  molar ratio 4/1/0.005

amount of support 40% in each element

the hue over the case where the tertiary amine prepared in the presence of the conventional catalyst (Comparative Examples 7 and 8) was used as the starting material.

EP 0 487 514 B1

Examples 11 and Comparative Example 9

A reaction of lauryl alcohol with ammonia was conducted in the presence of a Cu/Co/Pd catalyst. In this reaction, ammonia was blown into the system at a feed rate of 40 ℓ/hr. The reaction was monitored by measurement of an amine value and gas chromatography. The secondary amine thus prepared was purified by distillation and then converted into a quaternary ammonium salt. In Comparative Examples, the same reaction was conducted in the presence of a Cu/Ni/Pd catalyst system and a Cu/Ni catalyst system.

The results are shown in Table 5.

It was found from the results that as with the Cu/Ni/Pd catalyst system (Comparative Example 9), the present catalyst system enabled a secondary amine to be prepared through a reaction of lauryl alcohol with ammonia with high activity and selectivity by varying the ammonia feed rate. Further, it was found that the present catalyst system brought about a remarkable

Table 5

| catalyst composition* | reaction time (hr) | composition of reaction product (wt%) | | | hue of quaternary ammonium salt (Lovibond Red) |
|---|---|---|---|---|---|
| | | dilauryl-amine | tertiary-amine | Others | dilauryldimethyl-ammonium chloride |
| Ex. 11 — Cu/Co/Pd | 5 | 93.7 | 3.9 | 2.4 | 1.2 |
| Comp. Ex. 9 — Cu/Ni/Pd | 5 | 91.3 | 4.8 | 3.9 | 3.9 |

Note: * Cu/Co/Pd and Cu/Ni/Pd: molar ratio 3/1/0.05
amount of support 40%

reaction conditions:

alcohol: lauryl alcohol

amine: ammonia

amine feed rate: 40 ℓ/hr

reaction temperature: 180°C

amount of addition of catalyst: 0.3% based on alcohol

improvement in the hue of the quaternary ammonium salt over the Cu/Ni/Pd catalyst system (Comparative Example 9).

Example 12 and Comparative Example 10

A reaction of lauryl alcohol with stearylamine was conducted in the presence of a Cu/Co/Ru catalyst. The reaction was monitored by measurement of an amine value and gas chromatography. The secondary amine thus prepared was purified by distillation and then converted into a quaternary ammonium salt. In Comparative Example, the same reaction was conducted in the presence of a Cu/Ni/Ru catalyst system.

The results are shown in Table 6.

It was found from the results chat as with the Cu/Ni/Ru catalyst system (Comparative Example 10), the present catalyst system enabled the preparation of an amine through a reaction of lauryl alcohol with stearylamine with very high activity and high selectivity. Further, it was found that the present catalyst system brought about a remarkable improvement in the hue of the quaternary ammonium salt over the Cu/Ni/Ru catalyst system (Comparative Example 10).

Table 6

| | catalyst composition * | reaction time (hr) | composition of reaction product (wt%) | | | hue of quaternary ammonium salt (Lovibond Red) laurylstearyl-dimethylammonium chloride |
|---|---|---|---|---|---|---|
| | | | unreacted alcohol | N-lauryl-stearyl-amine | Others | |
| Ex. 12 | Cu/Co/Ru | 5 | 1.1 | 87.3 | 11.6 | 1.1 |
| Comp. Ex. 10 | Cu/Ni/Ru | 5 | 1.0 | 82.6 | 16.4 | 2.6 |

Note: * Cu/Co/Ru and Cu/Ni/Ru: molar ratio 3/1/0.005
amount of support 50%

reaction conditions:

alcohol: lauryl alcohol

amine: stearylamine

reaction temperature: 190°C

amount of addition of catalyst: 1.2% based on alcohol

Example 13 and Comparative Example 11

A reaction of 2-laurylcetyl alcohol with stearylamine was conducted in the presence of a Cu/Co/Pt catalyst. The reaction was monitored by measurement of an amine value and gas chromatography. The secondary amine thus prepared was purified by distillation and then converted into a quaternary ammonium salt. In Comparative Example, the same reaction was conducted in the presence of a Cu/Ni/Pt catalyst system.

The results are shown in Table 7.

13

It was found from the results that as with the Cu/Ni/Pt catalyst system (Comparative Example 11), the present catalyst system enabled the preparation of an amine through a reaction of 2-laurylcetyl alcohol with stearylamine with very high activity and high selectivity. Further, it was found that the present catalyst system brought about a remarkable improvement in the hue of the quaternary ammonium salt over the Cu/Ni/Pt catalyst system (Comparative Example 11).

Table 7

| | catalyst composition* | reaction time (hr) | composition of reaction product (wt%) | | | hue of quaternary ammonium salt (Lovibond Red) 2-laurylcetyl-stearyldimethyl-ammonium chloride |
| | | | unreacted alcohol | N,N-2-laurylcetyl-stearylamine | Others | |
|---|---|---|---|---|---|---|
| Ex. 13 | Cu/Co/Pt | 6 | 0.5 | 85.3 | 14.2 | 1.9 |
| Comp. Ex. 11 | Cu/Ni/Pt | 6 | 0.4 | 81.6 | 18.0 | 2.7 |

Note: * Cu/Co/Pt and Cu/Ni/Pt: molar ratio 3/2/0.01
amount of support 50%

reaction conditions:

alcohol: 2-laurylcetyl alcohol

amine: stearylamine

reaction temperature: 160°C

amount of addition of catalyst: 0.7% based on alcohol

reaction pressure: 30 atm (gauge pressure)

14

Example 14

The reaction mixture prepared in Example 1 was filtered to recover the catalyst therefrom, and the amination reaction was repeatedly conducted under the same conditions. The results are shown in Table 8.

Table 8

| number of runs | reaction time (hr) | composition of reaction production (wt%) | | | hue of quaternary ammonium salt (Lovibond Red) | |
|---|---|---|---|---|---|---|
| | | unreacted alcohol | lauryl-dimethyl-amine | Others | trimethyl-laurylammonium chloride | dimethyllauryl-benzylammonium chloride |
| 1 | 5 | 1.4 | 95.5 | 3.1 | 1.0 | 0.8 |
| 2 | 5 | 1.2 | 94.5 | 4.3 | 1.8 | 1.5 |
| 3 | 5 | 1.7 | 95.3 | 3.0 | 1.5 | 1.5 |
| 4 | 5 | 1.2 | 96.7 | 2.1 | 1.0 | 0.8 |
| 5 | 5 | 1.7 | 95.5 | 2.8 | 1.2 | 1.2 |

**Claims**

1. A process for preparing an N-substituted amine, which comprises the step of reacting an alcohol or an aldehyde with ammonia, a primary amine or a secondary amine at a temperature from 150 to 250°C and at a pressure from atmospheric pressure to 101.325 bar (100 atm gauge), with removal of water formed in the reaction, in the presence of a catalyst comprising (1) copper, (2) cobalt, and (3) a metal of the platinum VIII group.

15

2. A process as claimed in Claim 1, in which the molar ratio of the component (1) to (2) ranges from 10/90 to 99/1, and the molar ratio of the component (3) to the sum total of (1) and (2) ranges from 0.001 to 0.1.

**Patentansprüche**

1. Verfahren zum Herstellen eines N-substituierten Amins, umfassend den Schritt der Umsetzung eines Alkohols oder eines Aldehyds mit Ammoniak, einem primären Amin oder einem sekundären Amin bei einer Temperatur von 150 bis 250°C bei einem Druck von Atmosphärendruck bis 101,325 bar (100 Atmosphären-Meßanzeige) und Entfernung des in der Reaktion gebildeten Wassers in Gegenwart eines Katalysators, umfassend (1) Kupfer, (2) Kobalt und (3) ein Metall der Platingruppe VIII.

2. Verfahren nach Anspruch 1, worin das Molverhältnis der Komponente (1) zu (2) im Bereich von 10/90 bis 99/1 liegt, und das Molverhältnis der Komponente (3) zu der Gesamtsumme aus (1) und (2) im Bereich von 0,001 bis 0,1 liegt.

**Revendications**

1. Procédé pour la préparation d'une amine N-substituée comprenant la réaction d'un alcool ou d'un aldéhyde avec de l'ammoniac, une amine primaire ou une amine secondaire à une température comprise entre 150 et 250°C et à une pression comprise entre la pression atmosphérique et 101,325 bars (100 atmosphère manométrique), avec élimination de l'eau formée au cours de la réaction, en présence d'un catalyseur comprenant (1) du cuivre, (2) du cobalt, et (3) un métal appartenant au groupe VIII du platine.

2. Procédé selon la revendication 1 dans lequel le rapport molaire du constituant (1) au constituant (2) est compris entre 10/90 et 99/1 et le rapport molaire du constituant (3) à la somme des constituants (1) et (2) est compris entre 0, 001 et 0,1.